# EUROPEAN PATENT SPECIFICATION

(11) **EP 4 365 160 B1**
(45) Date of publication and mention of the grant of the patent: **04.02.2026**
(21) Application number: 23837928.3
(22) Date of filing: 18.05.2023
(51) Int. Cl.: C07C 51/46, C07C 51/44, C07C 51/377, C07C 51/48, C07C 57/04

(54) **METHOD FOR PREPARING ACRYLIC ACID**
VERFAHREN ZUR HERSTELLUNG VON ACRYLSÄURE
PROCÉDÉ DE PRÉPARATION D'ACIDE ACRYLIQUE

(30) Priority: 14.09.2022 KR 20220115926
(43) Date of publication of application: 08.05.2024
(73) Proprietor: LG Chem, Ltd., Seoul 07336 (KR)
(72) Inventor: LYU, Byeong Gil, Daejeon 34122 (KR); KIM, Mi Kyung, Daejeon 34122 (KR); SHIN, Dae Young, Daejeon 34122 (KR); JUNG, Da Bin, Daejeon 34122 (KR)
(74) Representative: Goddar, Heinz J.
(86) International application number: PCT/KR2023/006789
(87) International publication number: WO 2024/058340

(56) References cited:
- EP-B1- 3 044 198
- CN-B- 101 255 109
- JP-A- 2009 242 285
- JP-A- 2014 189 510
- JP-A- 2014 189 510
- JP-A- S6 087 241
- KR-A- 20160 122 749
- KR-A- 20170 113 177
- KR-A- 20220 078 233
- US-A- 4 554 054

## Description

### [Technical Field]

### CROSS-REFERENCE TO RELATED APPLICATION

The present application claims the benefit of priority to Korean Patent Application No. 10-2022-0115926, filed on September 14, 2022.

### Technical Field

The present invention relates to a method for preparing acrylic acid, and more particularly, to a method for effectively removing by-products while reducing loss of lactic acid and acrylic acid in preparing acrylic acid through a dehydration reaction of lactic acid.

### [Background Art]

Acrylic acid is used as a polymer raw material used in fibers, adhesives, paints, fiber processing, leather, construction materials, and the like, and its demand is expanding. In addition, the acrylic acid is also used as a raw material of an absorbent resin, and is widely used industrially for absorbent products such as paper diapers and sanitary napkins, water-retaining agents for agricultural and horticultural use, and waterstops for industrial use.

As a method for preparing acrylic acid according to the related art, a method of air oxidizing propylene is generally used, but this method is a method for preparing acrylic acid by converting propylene into acrolein by a gas-phase catalytic oxidation reaction, and subjecting the acrolein to a gas-phase catalytic oxidation reaction, and in this case, acetic acid is produced as a by-product, which is difficult to separate from acrylic acid. In addition, the method for preparing acrylic acid using propylene uses, as a raw material, propylene obtained by refining crude oil, which is a fossil resource, and has problems in terms of raw material costs or environmental pollution considering problems such as the recent rise in crude oil prices or global warming.

Accordingly, studies on a method for preparing acrylic acid from a carbon-neutral biomass raw material have been conducted. For example, there is a method for preparing acrylic acid (AA) through a gas-phase dehydration reaction of lactic acid (LA). This method is a method for preparing acrylic acid through an intramolecular dehydration reaction of lactic acid generally at a high temperature of 300°C or higher in the presence of a catalyst. A reaction product including acrylic acid is produced through the dehydration reaction of lactic acid, and unreacted lactic acid is included in the reaction product according to a conversion rate. In a case where unreacted lactic acid is included in the reaction product, the economic efficiency of the process may be improved only when the unreacted lactic acid is recovered in a separation process. However, an oligomerization reaction of the lactic acid proceeds rapidly at a high concentration and a high temperature, and it is difficult to recover the lactic acid.

JP2014189510 discloses a process to produce acrylic acid by dehydration of lactic acid, a condensing step, followed by an extraction step, wherein the extract is distilled in presence of the extraction solvent. CN101255109B discloses a process to produce acrylic acid by dehydration of lactic acid , a condensing step, followed by an extraction/azeotropic distillation in presence of an entrainer, wherein the extract is distilled in presence of the extraction solvent, wherein the reaction product is cooled in heat exchanger 1 and condensed in condenser 4 , and separated in the gas-liquid separator 5.

### [Disclosure]

### [Technical Problem]

In order to solve the problems mentioned in the background art, an object of the present invention is to provide a method capable of minimizing loss of lactic acid and acrylic acid by effectively separating lactic acid and acrylic acid from a reaction product produced in preparing acrylic acid through a dehydration reaction of lactic acid.

### [Technical Solution]

In one general aspect, a method for preparing acrylic acid includes: obtaining a reaction product including lactic acid, water, a light gas component, and acrylic acid by supplying a lactic acid aqueous solution to a reactor to allow a dehydration reaction to proceed; supplying the reaction product to a first cooling tower to separate the reaction product into a lower fraction containing acrylic acid and lactic acid and an upper fraction containing acrylic acid, water, and a light gas component; obtaining a lower fraction containing acrylic acid and water by supplying the upper fraction of the first cooling tower to a second cooling tower; obtaining an extract containing acrylic acid and an extraction solvent by supplying the lower fraction of the second cooling tower to an extraction column; obtaining a lower fraction containing acrylic acid and lactic acid by supplying the lower fraction of the first cooling tower and the extract to an azeotropic distillation column; and obtaining acrylic acid by supplying the lower fraction of the azeotropic distillation column to an acrylic acid recovery tower.

### [Advantageous Effects]

According to the method for preparing acrylic acid of the present invention, two cooling towers are used before distillation of the reaction product, such that energy costs required for purifying acrylic acid may be reduced, and at the same time, high-purity acrylic acid may be obtained.

In addition, a maximum content of lactic acid included in the reaction product is condensed and separated in the first cooling tower, such that loss of lactic acid in the first cooling tower may be prevented. In particular, lactic acid and acrylic acid condensed in the first cooling tower are separated in advance, and the separated lactic acid and acrylic acid are supplied to the azeotropic distillation column for purifying acrylic acid, such that loss of acrylic acid may be reduced, and high-purity acrylic acid may be obtained.

### [Description of Drawings]

FIG. 1 is a process flow chart according to a method for preparing acrylic acid in an exemplary embodiment of the present invention.
FIG. 2 is a process flow chart according to a method for preparing acrylic acid according to a comparative example for comparison with the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The term "stream" in the present invention may refer to a flow of a fluid in a process, and may also refer to a fluid itself flowing in a pipe. Specifically, the stream may refer to both a fluid itself flowing in a pipe connecting respective devices to each other and a flow of a fluid. In addition, the fluid may refer to a gas or liquid, and a case of the fluid including a solid component is not excluded.

Meanwhile, in the present invention, in devices such as a cooling tower, an extraction column, and a distillation column, the "lower portion" of the device refers to a point at a height of 95% to 100% from the top of the device to the bottom, unless otherwise specified, and specifically, may refer to the bottom (of the tower). Similarly, the "upper portion" of the device refers to a point at a height of 0% to 5% from the top of the device to the bottom, unless otherwise specified, and specifically, may refer to the top (of the tower).

In addition, unless otherwise specified, an operating temperature of the cooling tower in the present invention may refer to an operating temperature at the lower of the cooling tower, and an operating pressure of the cooling tower may refer to an operating pressure at the upper of the cooling tower.

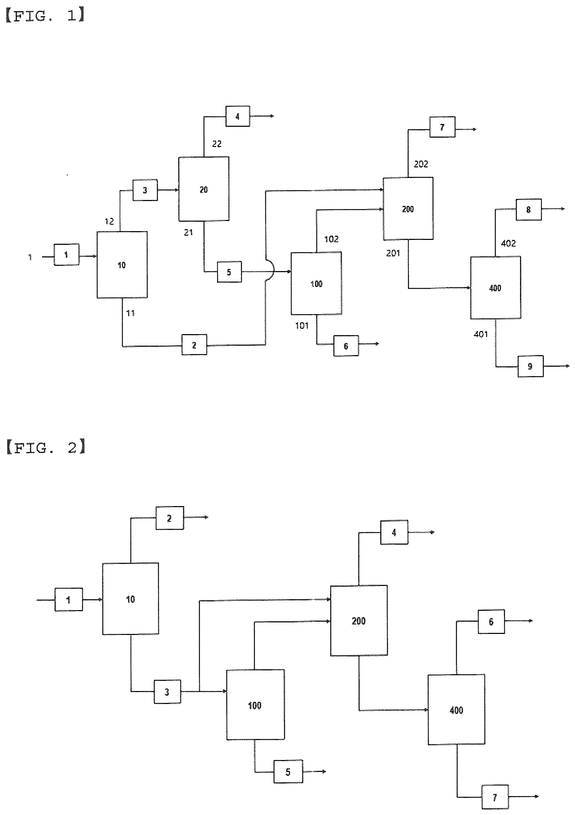

Hereinafter, each process that may be included in exemplary embodiments of the present invention will be described with reference to FIG. 1 and the like.

A method for preparing acrylic acid according to an exemplary embodiment of the present invention may include: obtaining a reaction product including lactic acid, water, a light gas component, and acrylic acid by supplying a lactic acid aqueous solution to a reactor to allow a dehydration reaction to proceed; supplying the reaction product to a first cooling tower to separate the reaction product into a lower fraction containing acrylic acid and lactic acid and an upper fraction containing acrylic acid, water, and a light gas component; obtaining a lower fraction containing acrylic acid and water by supplying the upper fraction of the first cooling tower to a second cooling tower; obtaining an extract containing acrylic acid and an extraction solvent by supplying the lower fraction of the second cooling tower to an extraction column; obtaining a lower fraction containing acrylic acid and lactic acid by supplying the lower fraction of the first cooling tower and the extract to an azeotropic distillation column; and obtaining acrylic acid by supplying the lower fraction of the azeotropic distillation column to an acrylic acid recovery tower.

First, the method for preparing acrylic acid according to an exemplary embodiment of the present invention may include obtaining a reaction product including lactic acid, water, a light gas component, and acrylic acid by supplying a lactic acid aqueous solution to a reactor to allow a dehydration reaction to proceed.

Specifically, as a method for preparing acrylic acid according to the related art, a method of air oxidizing propylene is generally used, but this method is a method for preparing acrylic acid by converting propylene into acrolein by a gas-phase catalytic oxidation reaction, and subjecting the acrolein to a gas-phase catalytic oxidation reaction, and in this case, acetic acid is produced as a by-product, which is difficult to separate from acrylic acid. In addition, the method for preparing acrylic acid using propylene uses, as a raw material, propylene obtained by refining crude oil, which is a fossil resource, and has problems in terms of raw material costs or environmental pollution considering problems such as the recent rise in crude oil prices or global warming.

In order to solve the problems of the method for preparing acrylic acid according to the related art, studies on a method for preparing acrylic acid from a carbon-neutral biomass raw material have been conducted. For example, there is a method for preparing acrylic acid (AA) through a gas-phase dehydration reaction of lactic acid (LA). This method is a method for preparing acrylic acid through an intramolecular dehydration reaction of lactic acid generally at a high temperature in the presence of a catalyst. A reaction product including acrylic acid is produced through the dehydration reaction of lactic acid, and unreacted lactic acid is included in the reaction product according to a conversion rate. In a case where unreacted lactic acid is included in the reaction product, the economic efficiency of the process may be improved only when the unreacted lactic acid is recovered in a separation process. However, an oligomerization reaction of the lactic acid proceeds rapidly at a high concentration and a high temperature, and it is difficult to recover the lactic acid.

Accordingly, in order to solve the problems in the related art, the present invention is intended to provide a method capable of improving a recovery rate of unreacted lactic acid and minimizing loss of acrylic acid that may occur during recovery of unreacted lactic acid by separating lactic acid from a reaction product including acrylic acid produced through a dehydration reaction of lactic acid in advance before a distillation process so that the time when lactic acid at a high concentration is exposed to a high temperature is shortened to prevent oligomerization of lactic acid.

That is, the entire amount of lactic acid is condensed and separated through a supercooling operation of the first cooling tower, but in this case, acrylic acid that may be condensed together with lactic acid is efficiently separated and purified, thereby providing a method capable of recovering or obtaining high-purity lactic acid and acrylic acid, respectively, without loss of lactic acid and acrylic acid.

According to an exemplary embodiment of the present invention, a reaction product including acrylic acid may be produced by supplying a lactic acid aqueous solution to a reactor in advance and allowing a dehydration reaction to proceed. In this case, the dehydration reaction may be performed as a gas phase reaction in the presence of a catalyst. For example, a concentration of lactic acid in the lactic acid aqueous solution may be 10 wt% or more, 20 wt% or more, or 30 wt% or more, and 40 wt% or less, 50 wt% or less, 60 wt% or less, or 70 wt% or less. When the lactic acid is present at a high concentration, oligomers such as dimers and trimers may be formed by an equilibrium reaction, and therefore, lactic acid may be used in the form of an aqueous solution at a concentration within the above range.

The reactor may include a reactor capable of dehydrating normal lactic acid, the reactor may include a reaction tube filled with a catalyst, and acrylic acid may be produced by dehydrating lactic acid by a gas-phase catalytic reaction while passing a reaction gas including volatile components of a lactic acid aqueous solution as a raw material through the reaction tube. In addition to lactic acid, the reaction gas may further include one or more diluent gases selected from water vapor, nitrogen, and air for concentration adjustment.

The operation of the reactor may be performed under normal lactic acid dehydration reaction conditions. In this case, an operating temperature of the reactor may refer to a set temperature of a heat medium or the like used for temperature control of the reactor.

The catalyst used in the dehydration reaction of lactic acid may include, for example, one or more selected from the group consisting of a sulfate-based catalyst, a phosphate-based catalyst, and a nitrate-based catalyst. As specific examples, the sulfate may include Na₂SO₄, K₂SO₄, CaSO₄, and Al₂(SO₄)₃, the phosphate may include Na₃PO₄, Na₂HPO₄, NaH₂PO₄, K₃PO₄, K₂HPO₄, KH₂PO₄, CaHPO₄, Ca₃(PO₄)₂, AlPO₄, CaH₂P₂O₇, and Ca₂P₂O₇, and the nitrate may include NaNO₃, KNO₃, and Ca(NO₃)₂. In addition, the catalyst may be supported on a support. Examples of the support include one or more selected from the group consisting of diatomite, alumina, silica, titanium dioxide, carbide, and zeolite.

The reaction product produced through the dehydration reaction of lactic acid may include water (H₂O), a light gas component, and lactic acid in addition to acrylic acid, which is a desired product. Here, the lactic acid may be unreacted lactic acid in the dehydration reaction of lactic acid.

The method for preparing acrylic acid through a dehydration reaction of lactic acid may secure raw material competitiveness compared to the method of air oxidizing propylene according to the related art and may solve the problem of environmental pollution, but a conversion rate of lactic acid is low, and various by-products are produced, resulting in a low yield of acrylic acid. Therefore, development of a process for improving economic efficiency is demanded. Accordingly, the present invention may provide a method for improving economic efficiency by reducing overall equipment costs and energy costs as well as obtaining high-purity acrylic acid without loss of acrylic acid and increasing a recovery rate of unreacted lactic acid.

The method for preparing acrylic acid according to an exemplary embodiment of the present invention may include supplying the reaction product to a first cooling tower 10 to separate the reaction product into a lower fraction containing acrylic acid and lactic acid and an upper fraction containing acrylic acid, water, and a light gas component.

Specifically, a reactor discharge stream 1 containing the reaction product is a gaseous stream, and the reactor discharge stream 1 may be supplied to the first cooling tower 10 to be cooled. That is, lactic acid having a relatively high boiling point in the gaseous reaction product supplied to the first cooling tower 10 is cooled and condensed, such that a liquid condensate may be formed.

That is, lactic acid included in the reaction product is separated in advance by cooling, such that deformation of lactic acid due to exposure to a high temperature, for example, oligomerization, may be prevented to increase a recovery rate of lactic acid, and the recovered lactic acid may be efficiently reused as a raw material for the dehydration reaction for preparing acrylic acid.

Here, in order to separate lactic acid without loss, it is important to control operating conditions of the first cooling tower 10. That is, when the operating condition of the first cooling tower 10 is a condition of excessive cooling of lactic acid, for example, when the cooling temperature is excessively decreased at the same pressure, the entire amount of lactic acid may be recovered, but acrylic acid may also be partially condensed together with lactic acid, which may cause loss of acrylic acid through the lower of the first cooling tower. Conversely, when the operating condition of the first cooling tower 10 is a condition of cooling a small amount of lactic acid, for example, when the cooling temperature is excessively increased at the same pressure, condensation of acrylic acid may be prevented, and thus, loss of acrylic acid may be prevented, but lactic acid may be partially uncondensed, which may cause loss of lactic acid through the upper of the first cooling tower 10.

In the present invention, the first cooling tower 10 is operated under a condition of excessive cooling of lactic acid so that a maximum amount of lactic acid included in the reaction product may be condensed, such that lactic acid and acrylic acid may be separated through the lower fraction of the first cooling tower 10. Through this, loss of lactic acid through the upper of the first cooling tower 10 due to non-condensation of lactic acid may be prevented. Meanwhile, acrylic acid contained in the lower fraction of the first cooling tower 10 is recovered in an azeotropic distillation column 200 and an acrylic acid recovery tower 400 described below, such that loss of acrylic acid may also be prevented. Therefore, loss of acrylic acid and lactic acid may be minimized, and at the same time, high-purity acrylic acid and lactic acid may be obtained and recovered, such that the quality of the product and economic efficiency may be improved.

To this end, an operating temperature of the first cooling tower 10 may be 110°C or higher and 140°C or less, and more specifically, 120°C or higher and 140°C or lower. The entire amount of lactic acid may be condensed within the above temperature range, and thus, loss of lactic acid may be minimized. In addition, acrylic acid may be condensed to the extent that acrylic acid may be separated with high purity in the acrylic acid recovery tower described below.

In addition, an operating pressure of the first cooling tower 10 may be 1 kg/cm² or more, 1.5 kg/cm² or more, or 2 kg/cm² or more, and 20 kg/cm² or less, 10 kg/cm² or less, or 5 kg/cm² or less. When the pressure is high, a volumetric flow rate is reduced, resulting in a reduction in cost of the cooling tower, but dimers of lactic acid and acrylic acid may be produced due to an increase in operating temperature of the cooling tower, and therefore, it is required to set an appropriate operating pressure at which a dimer is not produced.

When the operating conditions of the first cooling tower 10 are controlled to the operating temperature and the operating pressure within the above ranges, compositions of a lower discharge stream and an upper discharge stream of the first cooling tower 10 may be controlled, and through this, a composition of an aqueous solution stream discharged through the lower of the second cooling tower 20 may be easily controlled.

From this point of view, the upper fraction discharged from the first cooling tower 10 may not contain lactic acid, and even when lactic acid is included, lactic acid may be included in an amount of 2 wt% or less, and specifically, 1 wt% or less.

Meanwhile, acrylic acid contained in the upper fraction discharged from the first cooling tower 10 may be included in an amount of 80 wt% or less, 60 wt% or less, or 40 wt% or less, and 10 wt% or more, 20 wt% or more, or 30 wt% or more, with respect to acrylic acid introduced into the first cooling tower 10.

In addition, it is preferable that lactic acid included in the reaction product is condensed and separated as much as possible in the first cooling tower 10. Specifically, a ratio of a mass flow rate of lactic acid included in the lower fraction discharged from the first cooling tower 10 to a mass flow rate of lactic acid included in the reaction product may be 95 wt% to 99 wt%. That is, almost all of lactic acid included in the reaction product is condensed in the first cooling tower 10 and then separated. Therefore, in the present invention, the condition of excessive cooling of lactic acid means a cooling condition in which the ratio of the mass flow rate of lactic acid included in the lower fraction discharged from the first cooling tower 10 to the mass flow rate of lactic acid included in the reaction product satisfies the above range. Meanwhile, the separated lactic acid is distilled together with acrylic acid in the azeotropic distillation column 200 described below and then separated from water, and then separated into lactic acid and acrylic acid in the acrylic acid recovery tower 400.

Since the first cooling tower 10 is operated under the condition of excessive cooling of lactic acid, the upper fraction of the first cooling tower 10 may include acrylic acid, water, and a light gas component. Here, the light gas component is a component having a lower boiling point than water, and specifically, may include carbon monoxide, carbon dioxide, and acetaldehyde in addition to the diluent gas.

Subsequently, the upper fraction of the first cooling tower 10 may be supplied to the second cooling tower 20 as an upper discharge stream 12 of the first cooling tower. Meanwhile, the lower fraction of the first cooling tower 10 containing acrylic acid and lactic acid may be discharged as a lower discharge stream 11 of the first cooling tower, and the discharged lower fraction may be supplied to the azeotropic distillation column 200.

The upper discharge stream 12 of the first cooling tower 10 supplied to the second cooling tower 20 is additionally cooled, such that the upper discharge stream 12 may be separated into a lower fraction containing acrylic acid and water and an upper fraction containing a light gas component.

An operating temperature of the second cooling tower 20 may be 60°C or higher, 80°C or higher, or 100°C or higher, and 140°C or lower, 130°C or lower, or 120°C or lower, and an operating pressure of the second cooling tower 20 may be 1 kg/cm² or more, 1.5 kg/cm² or more, or 2 kg/cm² or more, and 20 kg/cm² or less, 10 kg/cm² or less, or 5 kg/cm² or less. As the operating conditions of the second cooling tower 20 are controlled to the operating temperature and the operating pressure within the above ranges, the composition of the light gas component separated through an upper discharge stream 24 of the second cooling tower 20 is controlled, such that loss of acrylic acid may be minimized, and at the same time, the light gas component containing a diluent gas and acetaldehyde may be removed out of the system.

In the method for preparing acrylic acid according to the related art, even when a cooling tower is used, the cooling tower is used for cooling the gaseous reaction product so that the gaseous reaction product is appropriately introduced into an acrylic acid purification process, and is not a cooling tower used for material separation. This is because it is difficult to recover a material with a desired purity when the material is separated by cooling. However, in the present invention, the cooling amount of the cooling tower is controlled to adjust the amount of components to be cooled, such that the material separation effect may also be obtained, in addition to cooling of the reaction product, which is the original object of the present invention.

Meanwhile, the lower fraction of the second cooling tower 20 may be discharged through a lower discharge stream 21 of the second cooling tower and may be supplied to an extraction column 100. The lower discharge stream 21 of the second cooling tower may contain acrylic acid that is not condensed in the first cooling tower 10. Obtaining an extract containing acrylic acid and an extraction solvent may be performed by the extraction process performed in the extraction column 100.

Specifically, the extraction column 100 removes most of the water contained in the lower discharge stream 21 of the second cooling tower without using a lot of energy and supplies the lower discharge stream 21 from which water is mostly removed to the azeotropic distillation column 200, such that energy used for azeotropic distillation in the azeotropic distillation column 200 described below may be reduced. In this respect, it is preferable that, in the extraction in the extraction column 100, the extraction solvent comes into contact with the stream supplied from the extraction column by a liquid-liquid contact method from the viewpoint of improving the energy efficiency of the entire process.

In this case, the extraction solvent may be a hydrocarbon-based solvent that may form an azeotrope with water and does not form an azeotrope with acrylic acid, but may sufficiently extract acrylic acid, and it is advantageous in the extraction process that the extraction solvent has a boiling point of 10 to 120°C. Specifically, the extraction solvent may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

In addition, as the extraction column 100, an extraction device based on a liquid-liquid contact method may be used. Non-limiting examples of the extraction device include a Karr reciprocating plate column, a rotary-disk contactor, a Scheibel column, a Kuhni column, a spray extraction tower, a packed extraction tower, a pulsed packed column, a mixer-settler bank, a mixer, and a centrifuge (centrifugal counter current extractor).

With such a method, an extract containing an extraction solvent and acrylic acid may be obtained, and the extract may be supplied to the azeotropic distillation column 200 as an upper discharge stream 102 of the extraction column. In addition, water may be recovered as a raffinate through the extraction process. The recovered raffinate may be discharged as a lower discharge stream 101 of the extraction column. As water is recovered in the extraction process as described above, the energy consumption may be significantly saved by reducing an operating load of a distillation process described below.

Subsequently, according to an exemplary embodiment of the present invention, the lower discharge stream 11 of the first cooling tower containing acrylic acid and lactic acid may be supplied to the azeotropic distillation column 200. A ratio of an amount of water to an amount of acrylic acid contained in the lower discharge stream 11 of the first cooling tower may be 2.5 to 4.0. When the lower discharge stream 11 of the first cooling tower is supplied to, for example, the extraction column 100, lactic acid is discharged together with water by the extraction process, such that loss of lactic acid is caused, or an additional device for separating lactic acid discharged together with water and energy required for this is required. Therefore, in the present invention, the lower discharge stream 11 of the first cooling tower is supplied to the azeotropic distillation column 200 together with the extract separated from the extraction column, such that loss of lactic acid and acrylic acid in the process of obtaining acrylic acid may be minimized. In addition, as the first cooling tower 10 is operated under the condition of excessive cooling of lactic acid, even when acrylic acid is discharged together with all or excessively condensed lactic acid, acrylic acid and lactic acid may be separated from each other in the acrylic acid recovery tower 400, such that loss of acrylic acid may be minimized.

Meanwhile, according to an exemplary embodiment of the present invention, the lower discharge stream 11 of the first cooling tower and the upper discharge stream 102 of the extraction column may be supplied to the azeotropic distillation column 200, and a distillation process for these streams may be performed. The distillation process in the azeotropic distillation column 200 for the stream supplied to the azeotropic distillation column 200 may be a process of separating the stream into an upper fraction containing water and an extraction solvent and a lower fraction containing acrylic acid and lactic acid by azeotropic distillation.

According to the present invention, it is advantageous in terms of process that the distillation in the azeotropic distillation column 200 is performed in the presence of an azeotropic solvent. Here, the azeotropic solvent is a hydrophobic solvent that may form an azeotrope with water and does not form an azeotrope with acrylic acid, and any hydrocarbon-based solvent that satisfies the physical properties may be used without limitation. In addition, the azeotropic solvent may have a boiling point lower than that of acrylic acid, and preferably, may have a boiling point of 10 to 120°C.

According to the present invention, the azeotropic solvent that satisfies the physical properties may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

In addition, the azeotropic solvent may be the same as or different from the extract solvent applied to the extraction column 100. However, considering production efficiency and the like according to a continuous process, the azeotropic solvent is preferably the same as the extraction solvent. As described above, when the same compound is used as the azeotropic solvent and the extraction solvent, at least a part of the azeotropic solvent distilled and recovered in the azeotropic distillation column 200 may be supplied to the extraction column 100 and used as a part of the extraction solvent.

When the azeotropic solvent is added to the azeotropic distillation column 200 as described above, the azeotrope of acrylic acid and water is broken. Accordingly, water and the azeotropic solvent used in the azeotropic distillation may form an azeotrope together and may be recovered through an upper fraction of the azeotropic distillation column 200. In addition, a lower fraction containing acrylic acid and lactic acid may be recovered through the lower of the azeotropic distillation column 200.

The upper fraction thus recovered of the azeotropic distillation column may be supplied to a layer separator through an upper discharge stream 202 of the azeotropic distillation column. The layer separator is a liquid-liquid layer separator and is a device for separating immiscible fluids using gravity or centrifugal force based on a density difference. A relatively light liquid may be separated through the upper of the layer separator, and a relatively heavy liquid may be separated through the lower of the layer separator. Specifically, the upper discharge stream 202 of the azeotropic distillation column supplied to the layer separator may be separated into an organic layer including an azeotropic solvent and a water layer including water.

In addition, the organic layer separated in the layer separator is discharged through a discharge stream of the layer separator, and the discharge stream of the layer separator containing an azeotropic solvent or an extraction solvent may be circulated to one or more of the extraction column and the azeotropic distillation column to be reused as an azeotropic solvent or an extraction solvent.

Meanwhile, the lower fraction of the azeotropic distillation column 200 containing acrylic acid and lactic acid may be supplied to the acrylic acid recovery tower 400 as a lower discharge stream 201 of the azeotropic distillation column. By distillation performed in the acrylic acid recovery tower 400, lactic acid may be separated through the lower of the acrylic acid recovery tower 400, and acrylic acid may be separated through the upper of the acrylic acid recovery tower 400. The lactic acid separated through the lower of the acrylic acid recovery tower 400 may be circulated to the reactor and may be reused as a raw material for the dehydration reaction.

Hereinabove, the method for preparing acrylic acid according to the present invention has been described and illustrated in the drawings. However, the description and the illustration of the drawings are for only essential components for understating the present invention, and processes and devices not separately described and illustrated may be properly applicable and used for implementing the method for preparing acrylic acid according to the present invention, in addition to the processes and devices described and illustrated in the drawings.

Hereinafter, the present invention will be described in more detail by Examples. However, the following Examples are provided for illustrating the present invention, and the scope of the present invention is not limited thereto.

### Examples

### Example 1

According to the process flow chart illustrated in FIG. 1, the acrylic acid producing process was simulated using Aspen Plus simulator available from Aspen Technology, Inc.

Specifically, 30 wt% of a lactic acid aqueous solution and nitrogen (N₂) as a diluent gas were supplied to a reactor, and a reaction product including lactic acid, water, a light gas component, and acrylic acid was produced through a dehydration reaction.

A reactor discharge stream containing the reaction product was supplied to a first cooling tower 10. In the first cooling tower 10, the reaction product was cooled and condensed, and then separated into a lower discharge stream 11 of the first cooling tower containing acrylic acid and lactic acid and an upper discharge stream 12 of the first cooling tower containing acrylic acid, water, and a light gas component. At this time, a lower operating temperature and an upper operating pressure of the first cooling tower 10 were controlled to 120°C and 1.9 kg/cm², respectively.

Subsequently, the lower discharge stream 11 of the first cooling tower was introduced into an azeotropic distillation column 200. Meanwhile, the upper discharge stream 12 of the first cooling tower was supplied to a second cooling tower 20 and then cooled and condensed, such that the upper discharge stream 12 of the first cooling tower was separated into a lower discharge stream 21 of the second cooling tower containing water and acrylic acid and an upper discharge stream 22 of the second cooling tower containing a light gas component including nitrogen. At this time, a lower operating temperature and an upper operating pressure of the second cooling tower 20 were controlled to 107°C and 1.3 kg/cm², respectively.

Meanwhile, the lower discharge stream 21 of the second cooling tower was supplied to an extraction column 100, acrylic acid was dissolved using toluene as an extraction solvent in the extraction column 100, and then, an extract containing acrylic acid and an extraction solvent was separated through an upper discharge stream 102 of the extraction column 100 and supplied to the azeotropic distillation column 200, and a lower discharge stream 101 of the extraction column 100 was discharged out of the system.

In addition, distillation was performed in the azeotropic distillation column 200 to which the lower discharge stream of the first cooling tower 10 and the upper discharge stream 102 of the extraction column 100 were supplied, such that a lower discharge stream 201 containing lactic acid and acrylic acid was obtained and then supplied to an acrylic acid recovery tower 400. Meanwhile, a stream containing water and an extraction solvent was discharged through the upper of the azeotropic distillation column 200. Subsequently, the stream containing water and an extraction solvent was supplied to a layer separator to separate the stream into water and an extraction solvent, water was discharged out of the system, and the extraction solvent was separately circulated to the extraction column 100 and the azeotropic distillation column 200.

Meanwhile, distillation was performed in the acrylic acid recovery tower 400, such that lactic acid was obtained through the lower of the acrylic acid recovery tower 400, and acrylic acid was obtained through the upper of the acrylic acid recovery tower 400.

In this case, the flow rate (kg/hr) and composition (wt%) of each component in each stream are shown in Table 1.

**[Table 1]**

| Component | Unit | 1 | 2 | 3 | 4 | 5 | 6 | 7 | 8 | 9 |
|---|---|---|---|---|---|---|---|---|---|---|
| Flow rate | | 11000 | 2755 | 8245 | 1023 | 7222 | 5330 | 1647 | 1910 | 590 |
| N2 | kg/hr | 1000 | 0 | 1000 | 1000 | 0 | 0 | 0 | 0 | 0 |
| Lactic acid | kg/hr | 600 | 585 | 15 | 0 | 15 | 14 | 0 | 5 | 582 |
| Water | kg/hr | 7480 | 1603 | 5877 | 22 | 5855 | 5812 | 1645 | 0 | 0 |
| Acrylic acid | kg/hr | 1920 | 567 | 1353 | 1 | 1352 | 4 | 1 | 1905 | 8 |

| Composition | | | | | | | | | | |
|---|---|---|---|---|---|---|---|---|---|---|
| N2 | wt% | 9% | 0% | 12% | 98% | 0% | 0% | 0% | 0% | 0% |
| Lactic acid | wt% | 5% | 2% | 0% | 0% | 0% | 0% | 0% | 0% | 99% |
| Water | wt% | 68% | 58% | 71% | 2% | 81% | 100% | 100% | 0% | 0% |
| Acrylic acid | wt% | 17% | 21% | 16% | 0% | 19% | 0% | 0% | 100% | 1% |

Referring to Table 1 and FIG. 1, as a result of comparing the mass flow rate of acrylic acid and lactic acid introduced into the first cooling tower 10 through the stream 1 and the mass flow rate of acrylic acid and lactic acid obtained through the upper and lower of the acrylic acid recovery tower 400, it could be confirmed that the recovery rate of lactic acid was 97%, and the recovery rate of acrylic acid was 99.2%.

### Example 2

According to the process flow chart illustrated in FIG. 1, the acrylic acid producing process was simulated using Aspen Plus simulator available from Aspen Technology, Inc.

Specifically, a process was performed in the same manner as that of Example 1, except that 40 wt% of a lactic acid aqueous solution was supplied to the reactor.

In this case, the flow rate (kg/hr) and composition (wt%) of each component in each stream are shown in Table 2.

**[Table 2]**

| Component | Unit | 1 | 2 | 3 | 4 | 5 | 6 | 7 | 8 | 9 |
|---|---|---|---|---|---|---|---|---|---|---|
| Flow rate | | 1100 | 5041 | 5959 | 1023 | 4936 | 4018 | 2399 | 1580 | 1980 |
| N2 | kg/hr | 1000 | 0 | 1000 | 1000 | 0 | 0 | 0 | 0 | 0 |
| Lactic acid | kg/hr | 2000 | 1936 | 34 | 0 | 34 | 32 | 0 | 17 | 1951 |
| Water | kg/hr | 6400 | 2366 | 4034 | 22 | 4012 | 3981 | 2397 | 0 | 0 |
| Acrylicacid | kg/hr | 1600 | 708 | 892 | 1 | 891 | 4 | 2 | 1583 | 29 |

| Composition | | | | | | | | | | |
|---|---|---|---|---|---|---|---|---|---|---|
| N2 | wt% | 9% | 0% | 17% | 98% | 0% | 0% | 0% | 0% | 0% |
| Lactic acid | wt% | 18% | 39% | 1% | 0% | 1% | 1% | 0% | 1% | 99% |
| Water | wt% | 53% | 47% | 68% | 2% | 81% | 99% | 100% | 0% | 0% |
| Acrylicacid | wt% | 15% | 14% | 15% | 0% | 18% | 0% | 0% | 99% | 1% |

Referring to Table 2 and FIG. 1, as a result of comparing the mass flow rate of acrylic acid and lactic acid introduced into the first cooling tower 10 through the stream 1 and the mass flow rate of acrylic acid and lactic acid obtained through the upper and lower of the acrylic acid recovery tower 400, it could be confirmed that the recovery rate of lactic acid was 97.6%, and the recovery rate of acrylic acid was 97.7%.

### Comparative Examples

### Comparative Example 1

According to the process flow chart illustrated in FIG. 2, the acrylic acid producing process was simulated using Aspen Plus simulator available from Aspen Technology, Inc.

In Comparative Example 1, a light gas component was separated using one cooling tower 10, a part of a lower discharge stream of the cooling tower 10 containing acrylic acid and lactic acid was supplied to an extraction column 100, and a remainder was introduced into an azeotropic distillation column 200, such that the same acrylic acid recovery rate as in Example 1 was realized.

As in Example 1, a reaction product was produced using a lactic acid aqueous solution in Comparative Example 1. Specifically, 30 wt% of a lactic acid aqueous solution and nitrogen (N₂) as a diluent gas were supplied to a reactor, and a reaction product including lactic acid, water, a light gas component, and acrylic acid was produced through a dehydration reaction.

The reaction product was supplied to the cooling tower 10, a light gas component such as nitrogen was separated through the upper, and a stream containing lactic acid, acrylic acid, and water was separated through the lower. At this time, a lower operating temperature and an upper operating pressure of the cooling tower 10 were controlled to 108°C and 1.3 kg/cm², respectively.

Half of the mass flow rate of the lower discharge stream of the cooling tower 10 was supplied to the extraction column 100, and the other half was supplied to the azeotropic distillation column 200.

Meanwhile, in the extraction column 100, acrylic acid was dissolved using toluene as an extraction solvent, and then an extract containing acrylic acid and an extraction solvent was separated through the upper discharge stream of the extraction column 100 and supplied to the azeotropic distillation column 200. Subsequently, distillation was performed in the azeotropic distillation column 200, and the upper discharge stream of the extraction column 100 was separated into a lower discharge stream containing lactic acid and acrylic acid and an upper discharge stream containing water and an extraction solvent. Meanwhile, the lower discharge stream of the azeotropic distillation column 200 containing lactic acid and acrylic acid was supplied to an acrylic acid recovery tower 400, and distillation was performed in the acrylic acid recovery tower 400, such that lactic acid was obtained through the lower of the acrylic acid recovery tower 400, and acrylic acid was obtained through the upper of the acrylic acid recovery tower 400.

The flow rate (kg/hr) and composition (wt%) of each component in each stream in Comparative Example 1 are shown in Table 3.

**[Table 3]**

| Component | Unit | 1 | 2 | 3 | 4 | 5 | 6 | 7 |
|---|---|---|---|---|---|---|---|---|
| Flow rate | | 11000 | 1023 | 9977 | 4281 | 3429 | 1910 | 356 |
| N2 | kg/hr | 1000 | 1000 | 0 | 0 | 0 | 0 | 0 |
| Lactic acid | kg/hr | 600 | 0 | 600 | 0 | 244 | 5 | 351 |
| Water | kg/hr | 7480 | 22 | 1458 | 4281 | 3177 | 0 | 0 |
| Acrylic acid | kg/hr | 1920 | 1 | 1919 | 0 | 9 | 1905 | 5 |

| Composition | | | | | | | | |
|---|---|---|---|---|---|---|---|---|
| N2 | wt% | 9% | 98% | 0% | 0% | 0% | 0% | 0% |
| Lactic acid | wt% | 5% | 0% | 6% | 0% | 7% | 0% | 99% |
| Water | wt% | 68% | 2% | 75% | 100% | 93% | 0% | 0% |
| Acrylic acid | wt% | 17% | 0% | 19% | 0% | 0% | 100% | 1% |

Referring to Table 3 and FIG. 2, as a result of comparing the mass flow rate (600 kg/hr) of lactic acid introduced through the stream No. 1 and the mass flow rate (351 kg/hr) of lactic acid recovered through the stream No. 7, it could be confirmed that the recovery rate of lactic acid was 58.5%.

### Comparative Example 2

According to the process flow chart illustrated in FIG. 2, the acrylic acid producing process was simulated using Aspen Plus simulator available from Aspen Technology, Inc.

Specifically, a process was performed in the same manner as that of Comparative Example 1, except that 40 wt% of a lactic acid aqueous solution was supplied to the reactor, and the same acrylic acid recovery rate (97.7%) as in Example 1 was realized.

The flow rate (kg/hr) and composition (wt%) of each component in each stream in Comparative Example 2 are shown in Table 4.

**[Table 4]**

| Component | Unit | 1 | 2 | 3 | 4 | 5 | 6 | 7 |
|---|---|---|---|---|---|---|---|---|
| Flow rate | | 11000 | 1023 | 9977 | 3662 | 3475 | 1580 | 1260 |
| N2 | kg/hr | 1000 | 1000 | 0 | 0 | 0 | 0 | 0 |
| Lactic acid | kg/hr | 2000 | 0 | 2000 | 0 | 754 | 17 | 1229 |
| Water | kg/hr | 6400 | 22 | 6378 | 3659 | 2719 | 0 | 0 |
| Acrylic acid | kg/hr | 1600 | 1 | 1599 | 3 | 2 | 1563 | 31 |

| Composition | | | | | | | | |
|---|---|---|---|---|---|---|---|---|
| N2 | wt% | 9% | 98% | 0% | 0% | 0% | 0% | 0% |
| Lactic acid | wt% | 18% | 0% | 20% | 0% | 22% | 1% | 98% |
| Water | wt% | 58% | 2% | 64% | 100% | 78% | 0% | 0% |
| Acrylic acid | wt% | 15% | 0% | 16% | 0% | 0% | 99% | 2% |

Referring to Table 4 and FIG. 2, as a result of comparing the mass flow rate (2,000 kg/hr) of lactic acid introduced through the stream No. 1 and the mass flow rate (1,229 kg/hr) of lactic acid recovered through the stream No. 7, it could be confirmed that the recovery rate of lactic acid was 61.5%.

## Claims

1. A method for preparing acrylic acid, the method comprising:
obtaining a reaction product including lactic acid, water, a light gas component, and acrylic acid by supplying a lactic acid aqueous solution to a reactor to allow a dehydration reaction to proceed;
supplying the reaction product to a first cooling tower to separate the reaction product into a lower fraction containing acrylic acid and lactic acid and an upper fraction containing acrylic acid, water, and a light gas component;
obtaining a lower fraction containing acrylic acid and water by supplying the upper fraction of the first cooling tower to a second cooling tower;
obtaining an extract containing acrylic acid and an extraction solvent by supplying the lower fraction of the second cooling tower to an extraction column;
obtaining a lower fraction containing acrylic acid and lactic acid by supplying the lower fraction of the first cooling tower and the extract to an azeotropic distillation column; and
obtaining acrylic acid by supplying the lower fraction of the azeotropic distillation column to an acrylic acid recovery tower.

2. The method of claim 1, wherein an operating temperature of the first cooling tower is 110°C to 140°C and an operating pressure of the first cooling tower is 1 kg/cm² to 20 kg/cm².

3. The method of claim 1, wherein an amount of lactic acid contained in an upper fraction discharged from the first cooling tower is 2 wt% or less.

4. The method of claim 1, wherein a ratio of a mass flow rate of lactic acid contained in the lower fraction discharged from the first cooling tower to a mass flow rate of lactic acid included in the reaction product is 95 wt% to 99 wt%.

5. The method of claim 1, wherein a ratio of an amount of water to an amount of acrylic acid contained in the lower fraction discharged from the first cooling tower is 2.5 to 4.0.

6. The method of claim 1, wherein a light gas component is separated through the upper of the second cooling tower.

7. The method of claim 1, wherein an operating temperature of the second cooling tower is 60°C to 140°C and an operating pressure of the second cooling tower 1 kg/cm² to 20 kg/cm².

8. The method of claim 1, wherein lactic acid is separated through the lower of the acrylic acid recovery tower, and acrylic acid is separated through the upper of the acrylic acid recovery tower.

9. The method of claim 8, wherein the lactic acid separated through the lower of the acrylic acid recovery tower is circulated to the reactor.

10. The method of claim 1, wherein an upper fraction of the azeotropic distillation column is supplied to a layer separator to separate water and an extraction solvent, and the separated extraction solvent is circulated to one or more of the extraction column and the azeotropic distillation column.

## Patentansprüche

1. Verfahren zur Herstellung von Acrylsäure, wobei das Verfahren umfasst:
Erhalten eines Reaktionsprodukts, das Milchsäure, Wasser, eine Leichtgaskomponente und Acrylsäure enthält, durch Zuführen einer wässrigen Milchsäurelösung zu einem Reaktor, um eine Dehydratisierungsreaktion ablaufen zu lassen;
Zuführen des Reaktionsprodukts zu einem ersten Kühlturm, um das Reaktionsprodukt in eine untere Fraktion, die Acrylsäure und Milchsäure enthält, und eine obere Fraktion, die Acrylsäure, Wasser und eine Leichtgaskomponente enthält, zu trennen;
Erhalten einer unteren Fraktion, die Acrylsäure und Wasser enthält, durch Zuführen der oberen Fraktion des ersten Kühlturms zu einem zweiten Kühlturm;
Erhalten eines Extrakts, der Acrylsäure und ein Extraktionslösungsmittel enthält, durch Zuführen der unteren Fraktion des zweiten Kühlturms zu einer Extraktionssäule;
Erhalten einer unteren Fraktion, die Acrylsäure und Milchsäure enthält, durch Zuführen der unteren Fraktion des ersten Kühlturms und des Extrakts zu einer azeotropen Destillationssäule; und
Erhalten von Acrylsäure durch Zuführen der unteren Fraktion der azeotropen Destillationssäule zu einem Acrylsäurerückgewinnungsturm.

2. Verfahren nach Anspruch 1, wobei eine Betriebstemperatur des ersten Kühlturms 110 °C bis 140 °C beträgt und ein Betriebsdruck des ersten Kühlturms 1 kg/cm² bis 20 kg/cm² beträgt.

3. Verfahren nach Anspruch 1, wobei eine Menge an Milchsäure, die in einer oberen Fraktion enthalten ist, die aus dem ersten Kühlturm ausgetragen wird, 2 Gew.-% oder weniger beträgt.

4. Verfahren nach Anspruch 1, wobei ein Verhältnis einer Massendurchflussrate von Milchsäure, die in der unteren Fraktion enthalten ist, die aus dem ersten Kühlturm ausgetragen wird, zu einer Massendurchflussrate von Milchsäure, die in dem Reaktionsprodukt enthalten ist, 95 Gew.-% bis 99 Gew.-% beträgt.

5. Verfahren nach Anspruch 1, wobei ein Verhältnis einer Menge an Wasser zu einer Menge an Acrylsäure, die in der unteren Fraktion enthalten ist, die aus dem ersten Kühlturm ausgetragen wird, 2,5 bis 4,0 beträgt.

6. Verfahren nach Anspruch 1, wobei eine Leichtgaskomponente durch den oberen Teil des zweiten Kühlturms getrennt wird.

7. Verfahren nach Anspruch 1, wobei eine Betriebstemperatur des zweiten Kühlturms 60 °C bis 140 °C beträgt und ein Betriebsdruck des zweiten Kühlturms 1 kg/cm² bis 20 kg/cm² beträgt.

8. Verfahren nach Anspruch 1, wobei Milchsäure durch den unteren Teil des Acrylsäurerückgewinnungsturms getrennt wird und Acrylsäure durch den oberen Teil des Acrylsäurerückgewinnungsturms getrennt wird.

9. Verfahren nach Anspruch 8, wobei die durch den unteren Teil des Acrylsäurerückgewinnungsturms getrennte Milchsäure zu dem Reaktor zirkuliert wird.

10. Verfahren nach Anspruch 1, wobei eine obere Fraktion der azeotropen Destillationssäule zu einem Schichtseparator zugeführt wird, um Wasser und ein Extraktionslösungsmittel zu trennen, und das getrennte Extraktionslösungsmittel zu einer oder mehreren der Extraktionssäule und der azeotropen Destillationssäule zirkuliert wird.

## Revendications

1. Procédé de préparation d'acide acrylique, le procédé comprenant :
obtenir un produit de réaction comprenant de l'acide lactique, de l'eau, un composant de gaz léger et de l'acide acrylique en fournissant une solution aqueuse d'acide lactique à un réacteur afin de permettre à une réaction de déshydratation de se faire ;
fournir le produit de réaction à une première tour de refroidissement pour séparer le produit de réaction en une fraction inférieure contenant de l'acide acrylique et de l'acide lactique et une fraction supérieure contenant de l'acide acrylique, de l'eau et un composant de gaz léger ;
obtenir une fraction inférieure contenant de l'acide acrylique et de l'eau en fournissant la fraction supérieure de la première tour de refroidissement à une deuxième tour de refroidissement ;
obtenir un extrait contenant de l'acide acrylique et un solvant d'extraction en fournissant la fraction inférieure de la deuxième tour de refroidissement à une colonne d'extraction ;
obtenir une fraction inférieure contenant de l'acide acrylique et de l'acide lactique en fournissant la fraction inférieure de la première tour d'extraction et l'extrait à une colonne de distillation azéotropique ; et
obtenir l'acide acrylique en fournissant la fraction inférieure de la colonne de distillation azéotropique à une tour de récupération d'acide acrylique.

2. Procédé selon la revendication 1, dans lequel une température de fonctionnement de la première tour de refroidissement est de 110 °C à 140 °C et une pression de fonctionnement de la première tour de refroidissement est de 1 kg/cm² à 20 kg/cm².

3. Procédé selon la revendication 1, dans lequel une quantité d'acide lactique contenu dans la fraction supérieure déchargée de la première tour de refroidissement est de 2 % en poids ou moins.

4. Procédé selon la revendication 1, dans lequel un rapport de débit massique d'acide lactique contenu dans la fraction inférieure déchargée de la première tour de refroidissement à un débit massique d'acide lactique inclus dans le produit de réaction est de 95 % en poids à 99 % en poids.

5. Procédé selon la revendication 1, dans lequel un rapport d'une quantité d'eau à une quantité d'acide acrylique contenus dans la fraction inférieure déchargée de la première tour de refroidissement est de 2,5 à 4,0.

6. Procédé selon la revendication 1, dans lequel un composant de gaz léger est séparé à travers la partie supérieure de la deuxième tour de refroidissement.

7. Procédé selon la revendication 1, dans lequel une température de fonctionnement de la deuxième tour de refroidissement est de 60°C à 140°C et une pression de fonctionnement de la deuxième tour de refroidissement est de 1 kg/cm² à 20 kg/cm².

8. Procédé selon la revendication 1, dans lequel l'acide lactique est séparé à travers la partie inférieure de la tour de récupération d'acide acrylique, et l'acide acrylique est séparé à travers la partie supérieure de la tour de récupération d'acide acrylique.

9. Procédé selon la revendication 8, dans lequel l'acide lactique séparé à travers la partie inférieure de la tour de récupération d'acide acrylique est acheminé au réacteur.

10. Procédé selon la revendication 1, dans lequel la fraction supérieure de la colonne de distillation azéotropique est fournie à un séparateur de couches pour séparer l'eau et un solvant d'extraction, et le solvant d'extraction est acheminé à l'une ou plusieurs d'entre la colonne d'extraction et la colonne de distillation azéotropique.
